# FASCICULE DE BREVET EUROPEEN

(11) **EP 2 328 883 B1**
(45) Date de publication et mention de la délivrance du brevet: **06.08.2014**
(21) Numéro de dépôt: 09747893.7
(22) Date de dépôt: 22.09.2009
(51) Int. Cl.: C07D 311/58, C07D 311/66, C07D 407/04

(54) **PROCEDE DE PREPARATION DE NEBIVOLOL**
VERFAHREN ZUR HERSTELLUNG VON NEBIVOLOL
METHOD FOR PREPARING NEBIVOLOL

(30) Priorité: 24.09.2008 FR 0856415
(43) Date de publication de la demande: 08.06.2011
(73) Titulaire: Zach System, 49240 Avrille (FR)
(72) Inventeur: DERRIEN, Yvon, F-49770 La Meignanne (FR); CHENARD, Eric, F-49000 Angers (FR); BURGOS, Alain, 69230 Saint Genis Laval (FR)
(74) Mandataire: Nevant, Marc
(86) Numéro de dépôt international: PCT/FR2009/051775
(87) Numéro de publication internationale: WO 2010/034927

(56) Documents cités:
- WO-A-2008/040528

## Description

La présente invention concerne un procédé de préparation de nebivolol et plus particulièrement un procédé amélioré pour la synthèse d'une alpha-halocétone de la formule 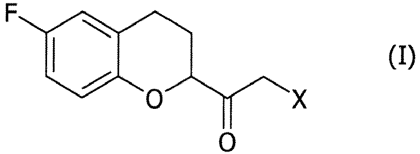 intermédiaire clé dans la préparation de nebivolol.

### DESCRIPTION DE L'ETAT DE LA TECHNIQUE

Le nebivolol (ci-après NBV) est un mélange de quantités égales de [2S[2R*[R[R*]]]]α,α'-[imino-bis(méthylène)]bis[6-fluoro-chroman-2-méthanol] (ci-après *d*-NBV) de la formule (IA) 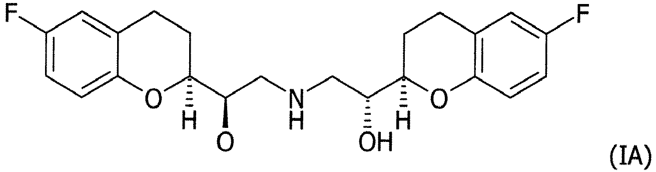 et de son énantiomère [2R[2S*[S[S*]]]] (ci-après /-NBV) de la formule (IB)

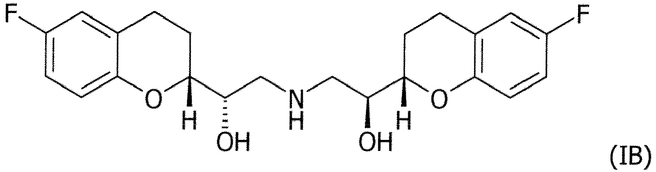

Le nebivolol est caractérisé par ses propriétés de blocage des récepteurs β-adrénergiques et est utile pour le traitement de l'hypertension artérielle essentielle. Il présente des propriétés basiques et peut être transformé en ses sels d'addition par traitement avec des acides appropriés. Le sel d'addition d'acide chlorhydrique est le produit mis sur le marché.

On sait dans la technique que la synthèse de structures moléculaires α,α'-[imino-bis(méthylène)]bis[chroman-2-méthanol] est un défi pour l'homme de l'art puisque les 4 atomes de carbone asymétriques produisent un mélange de 16 stéréoisomères (dans le cas de substitutions asymétriques) ou un mélange de 10 stéréoisomères (dans le cas de substitutions symétriques). On peut produire au total 10 stéréoisomères comme ceci apparaît par la présence de symétrie dans la structure du nebivolol

La littérature cite plusieurs procédés pour la préparation de nebivolol

Le brevet EP 145067 décrit un procédé de préparation de NBV qui comprend la synthèse de mélanges diastéréoisomères de dérivés de chromane-époxyde selon le schéma de synthèse ci-dessous

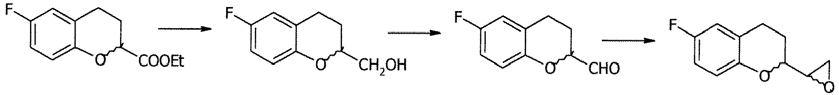

Le 6-fluorochromancarboxylate d'éthyle dérivé de l'estérification de l'acide correspondant est réduit avec du dihydrobis-(2-méthoxyéthoxy)-aluminate de sodium en alcool primaire ; le produit réagit avec du chlorure d'oxalyle et ensuite avec de la triéthylamine à -60°C pour fournir l'aldéhyde racémique correspondant qui est ensuite transformé en époxyde dans la forme d'un mélange de stéréoisomères (R,S), (S,R), (R,R) et (S,S).

Lesdits dérivés d'époxyde sont les intermédiaires clés du procédé.

Le brevet EP 334429 décrit principalement le même procédé de synthèse cité dans le brevet précédent et concerne particulièrement la préparation des isomères optiques uniques (R,S,S,S) et (S,R,R,R) de NBV.

L'acide 6-fluorochromancarboxylique est dans ce cas dédoublé en des énantiomères uniques par traitement avec de la (+)-déshydroabiétylamine. Lesdits énantiomères uniques sont transformés séparément en leurs époxydes correspondants résultant en un mélange de deux diastéréoisomères. Le schéma de synthèse suivant décrit par exemple la conversion du dérivé de S-acide.

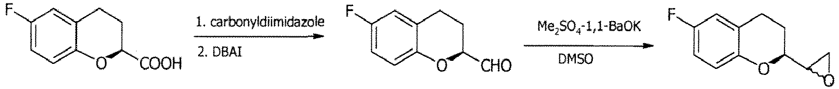

La demande de brevet international également en cours WO 2008/040528 au nom du même demandeur décrit un procédé amélioré pour la préparation de 6-fluorochromane-époxydes via une alpha-halocétone qui comprend la conversion d'un 6-fluoro-3,4-dihydro-2H-chromèn-2-carboxylate d'alkyle ou d'aryle en 2-halo-1-(6-fluoro-3,4-dihydro-2H-chromèn-2-yl)-éthanone ; la réduction dudit dérivé d'alpha-halocétone pour fournir le 2-halo-1-(6-fluoro-3,4-dihydro-2H-chromèn-2-yl)-éthanol correspondant ; et la cyclisation en présence d'une base pour fournir un dérivé d'époxyde dans la forme d'un mélange de quatre stéréoisomères.

Ladite étape de conversion est en particulier réalisée par réaction d'un chromancarboxylate d'alkyle ou d'aryle avec un ylure de sulfoxonium pour fournir l'ylure de cétosulfoxonium qui est transformé en une alpha-halocétone par réaction avec des acides halogénohydrogénés anhydres facultativement produits *in situ.*

La demande de brevet international WO 2008/010022 (Cimex Pharma and Industry of Zurich) décrit un procédé de fabrication de nebivolol racémique et de ses énantiomères purs et de sels pharmaceutiquement acceptables de ceux-ci.

Le procédé comprend, entre autres, la fourniture d'une alpha-halocétone racémique de la formule 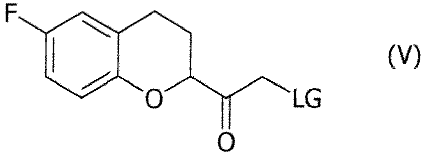 et sa conversion en 6-fluoro-chromane-époxyde ; en particulier ladite fourniture d'un composé de la formule V comprend
(1) la transformation d'un composé de la formule 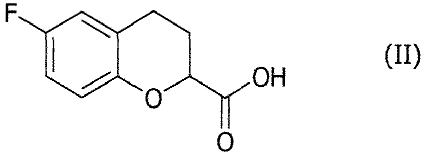 en un dérivé d'acide activé ;
(2) la réaction du dérivé d'acide activé avec un acide de Meldrum en présence d'une base pour fournir un composé de la formule 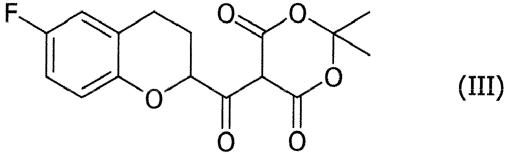
(3) la conversion du composé de la formule III en un composé de la formule 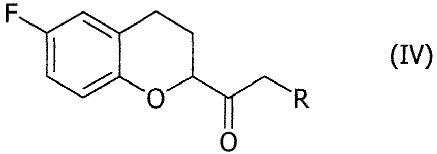 dans laquelle R est un atome d'hydrogène ou COOR' et dans laquelle R' est un groupe alkyle en C₁-C₆ ou aryle-alkyle en C₁ ; et
(4) l'halogénation du composé de la formule IV et la réalisation facultative d'une hydrolyse et d'une décarboxylation pour fournir le composé de la formule V.

Il s'avère selon l'art antérieur que les alpha-halocétones jouent un rôle essentiel dans la préparation de dérivés de 6-fluoro-chromane-époxyde et, à leur tour, de l'ingrédient pharmaceutique, le nebivolol.

### BUTS DE L'INVENTION

Il serait ainsi souhaitable d'étudier d'autres procédés de préparation de l'intermédiaire de la formule I dans une forme racémique ou de ses stéréoisomères uniques avec de bons rendements et dans des conditions plus favorables d'un point de vue d'une application à l'échelle industrielle.

### RESUME DE L'INVENTION

Nous avons maintenant trouvé de manière surprenante une autre synthèse facile et efficace de dérivés de 2-halo-1-(6-fluoro-3,4-dihydro-2H-chromèn-2-yl)-éthanone, intermédiaires clés dans la préparation de nebivolol, qui permet de supprimer les désavantages des procédés décrits dans l'art antérieur.

### DESCRIPTION DETAILLEE DE L'INVENTION

Un premier objet de la présente invention est par conséquent un procédé de préparation d'un composé de la formule 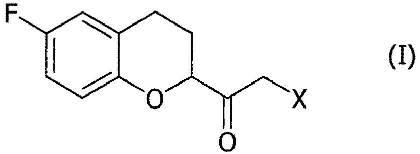 dans laquelle X est un atome d'halogène, qui comprend la réaction d'un composé de la formule 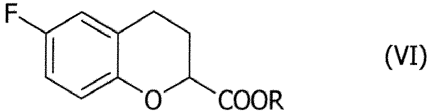 dans laquelle R est un groupe alkyle en C₁-C₆ ; avec un dérivé d'halométhyllithium.

Les composés de la formule VI sont des intermédiaires connus dans la préparation de NBV, dont la préparation est largement décrite dans la technique, se conférer par exemple aux brevets EP 145067 et EP 334429 cités ci-dessus.

L'halométhyllithium selon l'invention peut être représenté par la formule

Li-CH₂-X

dans laquelle X est défini ci-dessus ; et peut être préparé par réaction d'un composé d'organolithium et d'un di-halométhane.

Ledit composé d'organolithium et ledit di-halométhane sont dans un mode de réalisation préféré de l'invention ajoutés à un solvant de réaction et le corps réagissant d'halométhyllithium est formé dans le système réactionnel.

L'halométhyllithium préféré est le chlorométhyllithium et le bromométhyllithium, le premier étant encore mieux préféré.

Le composé d'organolithium préféré est le méthyllithium, le n-butyllithium et le sec-butyllithium, le n-butyllithium étant encore mieux préféré.

Le di-halométhane préféré utilisé dans l'invention est le bromochlorométhane, le dibromométhane et le chloroiodométhane, le premier étant celui que l'on préfère le plus.

Puisque l'on sait dans la technique que les dérivés d'halométhyllithium sont thermiquement instables, il est préférable de dissoudre préalablement un composé ester de la formule VI et un dihalométhane dans un solvant et d'ajouter ensuite le composé d'organolithium.

Le solvant préféré de l'invention est un solvant de type éther, tel que le tétrahydrofuranne, le diéthyléther, le tert-butylméthyléther et semblables. On peut utiliser dans l'objet de la réaction de l'invention un mélange de solvant de type éther et de solvants non polaires, tels que le toluène, l'hexane et semblables.

L'addition du réactif d'halométhyllithium au composé de la formule VI est réalisée en général à une température de -100°C à 0°C. La réaction est de préférence réalisée dans un intervalle de -85°C à -50°C.

Il est en général préférable, lorsque la réaction est achevée, de traiter le mélange réactionnel obtenu avec une solution aqueuse de chlorure d'ammonium, une solution de tampon de phosphate, de l'eau ou un acide, de préférence un acide faible, tel que l'acide acétique et semblables.

La quantité de composé d'organolithium et de dihalométhane utilisée dans l'invention n'est pas particulièrement critique. On utilise de préférence le substrat d'ester, le corps réagissant de dihalométhane et le composé d'organolithium dans un rapport molaire d'environ 1:2:2.

On indique dans la présente invention sous le terme halogène un atome de fluor, de chlore, de brome et d'iode.

X est de préférence un atome de chlore ou de brome, où l'on préfère encore mieux un atome de chlore.

Un autre objet de la présente invention est un procédé de préparation d'un composé de la formule 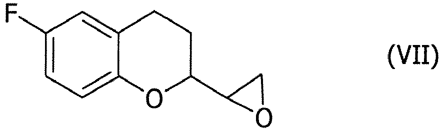 qui comprend une conversion d'un composé de la formule VII en un composé de la formule I selon ce qui est cité ci-dessus.

Un autre objet de la présente invention est un procédé de préparation d'un composé de la formule 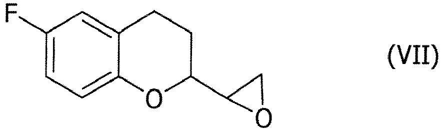 selon ce qui est cité ci-dessus comprenant de plus
a. la réduction d'un composé de la formule I pour fournir un composé de la formule 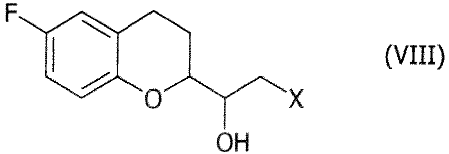
b. la réaction dudit composé de la formule VIII avec une base pour fournir le composé époxyde de la formule VII.

La réduction d'un composé de la formule I pour fournir un composé de la formule VIII (étape a) est réalisée selon des techniques connues.

Ladite réduction est dans un mode de réalisation de l'invention réalisée selon la demande de brevet international également en cours WO 2008/040528.

La réaction est de préférence réalisée en faisant réagir un composé de la formule I avec du borohydrure de sodium en présence d'un solvant alcoolique, facultativement mélangé avec de l'eau. Le solvant préféré est l'éthanol.

La réaction d'un composé de la formule VIII pour fournir un composé de la formule VII (étape b) est réalisée en présence d'une base selon des techniques connues. Ladite réaction de cyclisation est réalisée à nouveau dans un mode de réalisation de l'invention selon la demande de brevet international également en cours WO 2008/040528.

La cyclisation est de préférence réalisée en faisant réagir un composé de la formule VIII avec des alcoxydes ou des hydroxydes alcalins en présence de solvants alcooliques ou d'éthers facultativement en mélange.

Un mode de réalisation préféré de l'invention consiste en ce que la réaction est réalisée avec une base, telle que le t-butoxyde de potassium, en présence d'un mélange d'isopropanol/THF.

La réaction est sinon réalisée avec une base, telle que l'hydroxyde de sodium, en présence d'isopropanol.

Dans un autre mode de réalisation de l'invention, un composé de la formule VII est préparé par une procédure dans seulement un réacteur (de type « one-pot ») en partant d'un dérivé d'ester de la formule VI.

Ladite procédure comprend la réaction du substrat d'ester avec un dérivé d'halométhyllithium et une réduction/cyclisation *in situ* pour fournir directement des composés époxyde de la formule VII.

Le mélange réactionnel provenant de l'addition du dérivé d'halométhyllithium au composé de la formule VI est dans la pratique réduit *in situ* pour fournir une halohydrine de la formule VIII qui est à son tour cyclisée en présence d'une base.

Un autre objet de la présente invention est par conséquent un procédé de préparation d'un composé de la formule 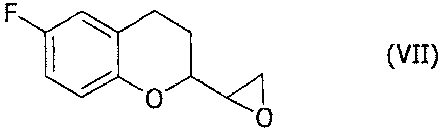 qui comprend la réaction d'un composé de la formule 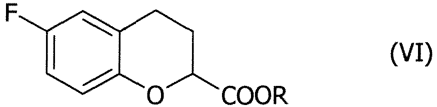 dans laquelle R est défini ci-dessus ; avec un dérivé d'halométhyllithium pour fournir un mélange réactionnel ; la réduction *in situ* dudit mélange réactionnel ; et la cyclisation en présence d'une base.

Ledit mélange réactionnel est dans un mode de réalisation préféré de l'invention réduit *in situ* avec un réactif de borane ; l'alcoxyborohydrure de lithium ainsi obtenu se transpose *in situ* et est hydrolysé en halohydrine qui est, à son tour, cyclisée en présence d'alcoxydes ou d'hydroxydes alcalins.

La réduction *in situ* selon l'invention est réalisée de préférence avec BH₃ dans THF.

Les inventeurs ont observé que le mélange réactionnel provenant de l'addition du dérivé d'halométhyllithium au composé de la formule VI comprend, comme entité chimique principale, un composé de la formule 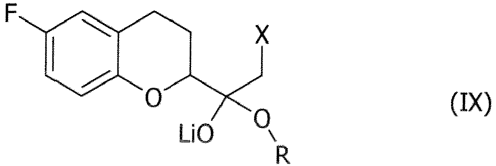 dans laquelle X et R sont définis ci-dessus ; lequel est soumis à la réduction *in situ* selon l'invention.

Il est ainsi évident que l'objet du procédé de l'invention constitue une alternative de synthèse efficace et économique, appropriée pour une production industrielle pour la préparation de chromane-époxydes ; de plus, la disponibilité des matières premières utilisées avec le nombre réduit d'étapes de synthèse et les rendements presque quantitatifs obtenus fournissent des avantages notables en termes de coût et d'efficacité du procédé. L'efficacité des réactions chimiques dans seulement un réacteur (réaction de type « one-pot ») est de plus bien souhaitée par les chimistes puisque l'on évite un long procédé de séparation et une purification des composés chimiques intermédiaires, ce qui fait gagner du temps et des matières premières tout en augmentant le rendement chimique.

Le procédé de conversion d'un ester en une alpha-halocétone et la conversion de type « one-pot » d'ester en époxyde selon l'invention sont de plus stéréoconservateurs pour des substrats munis de centres chiraux.

Le procédé de l'invention peut ainsi être appliqué aux esters optiquement actifs de la formule VI, lesquels peuvent être obtenus par estérification de l'acide optiquement actif correspondant ou sinon par chromatographie chirale selon des techniques connues.

Il est par conséquent clair pour l'homme de l'art que l'objet du procédé de l'invention mène à la préparation d'alpha-halocétone de la formule I enrichie en énantiomère et aux dérivés d'époxyde dans une forme racémique comprenant un mélange de deux diastéréoisomères, se conférer aux schémas ci-dessous : 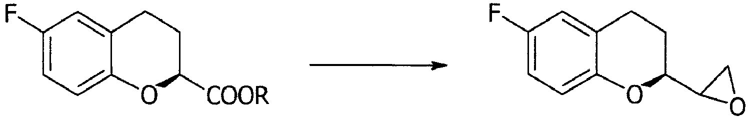 ou

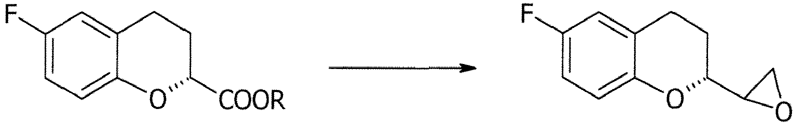

Comme connu, lesdits dérivés d'époxyde partiellement dédoublés sont des intermédiaires clés dans la préparation de NBV.

Un autre objet de l'invention est un procédé de synthèse de nebivolol caractérisé par le fait que la préparation d'un composé de la formule 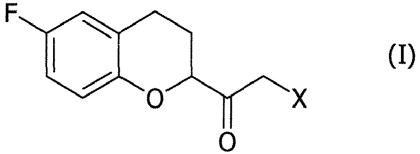 dans laquelle X est un atome d'halogène ; comprend la réaction d'un composé de la formule 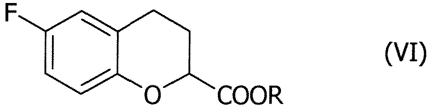 dans laquelle R est un groupe alkyle en C₁-C₆ ; avec un dérivé d'halométhyllithium.

Un autre objet de la présente invention est un procédé de synthèse de nebivolol qui comprend une conversion de type « one-pot » d'un composé de formule VI en un composé de formule VII selon ce qui est cité ci-dessus.

Un mode de réalisation pratique de l'objet du procédé de la présente invention comprend la conversion d'un 6-fluorochromancarboxylate de la formule VI en une alpha-halocétone de la formule I par addition d'un dérivé d'halométhyllithium ; ladite alpha-halocétone de la formule I est réduite en une halohydrine de la formule VIII et est cyclisée en un dérivé d'époxyde de la formule VII en présence d'une base.

Un mode de réalisation pratique préféré de l'objet du procédé de la présente invention comprend la conversion d'un 6-fluorochromancarboxylate d'alkyle en C₁-C₆ de la formule VI en l'alpha-chlorocétone correspondante de la formule I par addition d'un dérivé de chlorométhyllithium, obtenu de préférence *in situ* par réaction de bromochlorométhane ou d'iodochlorométhane avec un dérivé d'organolithium, tel que le n-butyllithium ; ladite alpha-chlorocétone de la formule I est réduite en une chlorohydrine de la formule VIII au moyen d'une réaction avec du borohydrure de sodium en présence d'un solvant alcoolique et est cyclisée en un dérivé d'époxyde de la formule VII par réaction avec des alcoxydes ou des hydroxydes alcalins en présence de solvants alcooliques ou d'éthers facultativement en mélange.

Un autre mode de réalisation pratique de l'objet du procédé de la présente invention comprend la conversion de type « one-pot » d'un 6-fluorochromancarboxylate de la formule VI en un dérivé d'époxyde de la formule VII par addition d'un dérivé d'halométhyllithium ; la réduction *in situ* du mélange réactionnel ainsi obtenu ; et la cyclisation en présence d'une base.

Un autre mode de réalisation pratique préféré de l'objet du procédé de la présente invention comprend la conversion d'un 6-fluorochromancarboxylate d'alkyle en C₁-C₆ de la formule VI en un dérivé d'époxyde de la formule VII par addition d'un dérivé de chlorométhyllithium obtenu de préférence *in situ* par réaction de bromochlorométhane ou d'iodochlorométhane avec un dérivé d'organolithium, tel que le n-butyllithium, pour fournir un mélange réactionnel ; ledit mélange est réduit *in situ* avec BH₃ en présence de THF et est cyclisé en un dérivé d'époxyde de la formule VII par réaction avec des alcoxydes ou des hydroxydes alcalins.

L'invention sera maintenant mieux illustrée par les exemples suivants.

Dans les exemples tous les pourcentages sont donnés en poids, la température est en degré celcius et la pression est la pression atmosphérique, sauf indication contraire.

### Exemple 1

### Synthèse d'acide 6-fluoro-4-oxo-4H-1-benzopyran-2-carboxylique

On a lentement ajouté à une solution de 52 ml de méthoxyde de sodium à 30 % dans du méthanol (0,30 mol) à 5-10°C une solution de 20 g de 5'-fluoro-2'-hydroxyacétophénone (13 mol) dans 100 ml de THF. On a ensuite laissé la température atteindre 15°C et on a ajouté 22 g d'oxalate de diéthyle (0,15 mol) entre 15 et 25°C pour fournir une solution complète. On a surveillé la progression de la réaction par CCM jusqu'à ce que la matière première de départ soit d'au plus 5 %. On a ajusté le mélange à pH 1-2 par addition de 20 ml d'HCl à 36 % (0,24 mol) et 100 ml d'eau entre 5 et 15°C. On a éliminé par filtration le résidu solide qui est formé, on a ensuite éliminé la couche aqueuse par décantation. On a ensuite lavé la couche organique avec 40 ml d'une solution aqueuse de NaCl à 15 % et on l'a concentrée sous pression réduite à 40-50°C pour fournir une huile jaune.

On a dissous cette huile résiduelle dans 100 ml d'acide acétique glacial et 100 ml d'eau sous reflux pendant 17-20 h. On a, après refroidissement à 15-20°C, filtré la suspension du composé indiqué dans le titre pour fournir après séchage 20 g d'une matière solide blanche ou beige (rendement : 75 %).

On peut obtenir une seconde récolte après concentration de la liqueur-mère et après chauffage du résidu dans 50 ml d'acide acétique glacial et 50 ml d'eau sous reflux pendant 20 h pour hydrolyser l'ester afin de fournir un lot supplémentaire du composé indiqué dans le titre (2-3 g).

### Exemple 2

### Synthèse de 6-fluoro-1-benzopyran-2-carboxylate de méthyle

On a hydrogéné sous 4 bars d'hydrogène dans 200 ml de THF 20 g d'acide 6-fluoro-4-oxo-4H-1-benzopyran-2-carboxylique (96 mmol) avec 2 g d'acide méthanesulfonique (20 mmol) et 2 g de Pd à 5 % sur du charbon actif(type Escat 112, Engelhard) à 45-55°C jusqu'à ce que l'on n'ait plus observé de consommation d'hydrogène. On a pu surveiller la progression de la réaction par CCM. On a ensuite éliminé le catalyseur par filtration et on a concentré le mélange réactionnel jusqu'à 40 ml sous pression réduite. On a ensuite ajouté 100 ml de méthanol et on a agité le mélange (solution) à 20-25°C jusqu'à ce que l'on a détecté moins de 1 % d'acide 6-fluoro-3,4-dihydro-2H-chromèn-2-carboxylique (CCM). On a ensuite concentré la solution sous pression réduite et on a dissous le résidu dans 60 ml de THF, on a ensuite concentré sous pression réduite pour éliminer l'eau par distillation azéotrope afin de fournir 20 g du composé indiqué dans le titre (rendement : 100 %).
δ_{H} (400 MHz ; CDCl₃) 6,89-6,79 (2H, m, Ar), 6,77-6,76-6,72 (1H, m, Ar), 4,73-4,69 (1H, m), 3,79 (3H, s), 2,87-2,69 (2H, m), 2,31-2,12 (2H, m).

### Exemple 3

### Synthèse de 2-chloro-1-(6-fluoro-1-benzopyran-2-yl)-éthanone

On a ajouté à température ambiante 1,2 g de bromochlorométhane (9,2 mmol) à une solution de 1 g de 6-fluoro-1-benzopyran-2-carboxylate de méthyle (4,7 mmol) brut dans 15 ml de THF. On a ensuite refroidi la solution à -80/-85°C et on a lentement ajouté 4,6 ml de n-BuLi 2,5 M dans de l'hexane (9,2 mmol) pour maintenir la température interne entre -75°C et -80°. On a surveillé la progression de la réaction par CCM. On a ensuite acidifié la solution par addition de 1 ml d'acide acétique glacial dans 2 ml de THF entre -70°C et -80°C. On a ensuite ajouté 5 ml d'eau à 0°C et on a ensuite éliminé la couche aqueuse après décantation. On a concentré la couche organique sous pression réduite pour fournir le composé indiqué dans le titre (1,05 g) dans la forme d'une huile jaune (titration par RMN : environ 80 % poids/poids).
δ_{H} (400 MHz ; CDCl₃) 6,86-6,83 (2H, m, Ar), 6,80-6,75 (1H, m, Ar), 4,69-4,65 (1H, m), 4,63 (1H, d, J 16,8), 4,47 (1H, d, J 16,8), 2,91-2,72 (2H, m), 2,34-2,26 (1H, m), 2,13-2,03 (1H, m) ; *m*/*z* (EI) 228,035339 (M⁺ . C₁₁H₁₀ClFO₂ exige 228,03551).

### Exemple 4

### Synthèse de 2-chloro-1-(6-fluoro-1-benzopyran-2-yl)-éthanol

On a refroidi à 0°C sous azote une solution agitée de 2-chloro-1-(6-fluoro-1-benzopyran-2-yl)-éthanone (0,33 g, 1,28 mmol, 88,4 % A) dans de l'éthanol (2,5 ml). On a ajouté NaBH₄ (60,1 mg, 1,59 mmol) à la solution et on a agité le mélange réactionnel pendant 2 h. Après avoir vérifié par CPG que le produit de départ a disparu, on a dilué le mélange avec de l'eau déminéralisée (7 ml) et du dichlorométhane (7 ml) et les phases se sont séparées. On a séché la strate organique sous sulfate de sodium anhydre, on l'a filtrée et concentrée sous vide pour fournir du 2-chloro-1-(6-fluoro-1-benzopyran-2-yl)-éthanol brut dans la forme d'un mélange de diastéréoisomères 54:46 (0,30 g, rendement 70 %, 67,9 % A).
δ_{H} (400 MHz ; CDCl₃) 6,83-6,70 (6H, m, Ar), 4,21-4,16 (1H, m), 4,02-3,96 (1H, m), 3,94-3,88 (3H, m), 3,86-3,77 (2H, m), 3,74-3,68 (1H, m), 2,97-2,74 (4H, m), 2,30-2,21 (2H, b, -OH), 2,29-2,22 (1H, m), 2,02-1,96 (2H, m), 1,89-1,78 (1H, m) ; *m*/*z* (EI) 230,050989 (M⁺ .C₁₁H₁₂ClFO₂ exige 230,05067).

### Exemple 5

### Synthèse de 6-fluoro-2-oxyranyl-1-benzopyrane

On a dissous dans i-PrOH (25 ml) sous azote du 2-chloro-1-(6-fluoro-1-benzopyran-2-yl)-éthanol (2,5 g, 9,20 mmol, 84,9 % A) et on a refroidi le mélange réactionnel à 0°C. On a ajouté à la solution une solution aqueuse 2M de NaOH (12,5 ml) en 5 min et on a agité la réaction pendant 1 h 30 min. On a ensuite dilué le mélange réactionnel avec du toluène (50 ml) et on a corrigé le pH avec de l'acide acétique (0,12 g). On a de plus ensuite ajouté au mélange du toluène (50 ml) et de l'eau déminéralisée (10 ml) et les phases se sont séparées après extraction. On a ensuite lavé les phases organiques recueillies avec de l'eau déminéralisée (50 ml). On a ensuite rendu la phase du toluène anhydre par distillation azéotrope et on l'a concentrée jusqu'à l'état sec dans un évaporateur rotatif pour fournir du 6-fluoro-2-oxyranyl-1-benzopyrane dans la forme d'un mélange de diastéréoisomères 52:48 (2,0 g, rendement 96 %, 86,1 % A).
Diast. RR,SS : δ_{H} (400 MHz ; CDCl₃) 6,81-6,72 (3H, m), 3,88-3,82 (1H, m), 3,21-3,17 (1H, m), 2,89-2,76 (4H, m), 2,1-2,00 (1H, m), 1,97-1,87 (1H, m) ; Diast. SR,SR : δ_{H} (400 MHz ; CDCl₃) 6,84-6,73 (3H, m), 3,87-3,81 (1H, m), 3,15-3,10 (1H, m), 2,91-2,78 (4H, m), 2,18-2,10 (1H, m), 1,96-1,84 (1H, m).

### Exemple 6

### Synthèse de 2-chloro-1-((R)-6-fluoro-1-benzopyran-2-yl)-éthanone

On a ajouté à température ambiante 2,5 g de bromochlorométhane (18,8 mmol) à une solution de 2,0 g de (R)-6-fluoro-1-benzopyran-2-carboxylate de méthyle obtenu après séparation du mélange racémique correspondant par une colonne de chromatographie chirale (9,4 mmol, 96,6 % A, ee > 99 %) dans 40 ml de THF. On a ensuite refroidi la solution à -80/-85°C et on a lentement ajouté 7,7 ml de n-BuLi 2,5 M dans de l'hexane (19 mmol) pour maintenir la température interne à de -75°C à -80°C.

On a surveillé la progression de la réaction par CCM. On a ensuite acidifié la solution par addition de 2 ml d'acide acétique glacial dans 2 ml de THF à de -70°C à -80°C. On a ensuite ajouté 10 ml d'eau à -20/0°C et on a ensuite éliminé la couche aqueuse après décantation. On a concentré la couche organique sous pression réduite pour fournir le composé indiqué dans le titre (2,2 g) dans la forme d'une matière solide beige (ee : 97,2 %, 94,8 % A).

On a ensuite facultativement fait recristalliser la matière solide dans un mélange d'acétate d'éthyle et d'hexane pour fournir 1,73 g de 2-chloro-1-((R)-6-fluoro-1-benzopyran-2-yl)-éthanone dans la forme d'une matière solide beige (rendement 80 %, ee : 99 %, 97,6 % A, [α]_{D} : -27°).
CHIRAL OB-H 250*4,6 mm particules 5 µm, N-heptane -IPOH (95 V-5 V), 200 nm, énantiomère (R) TR = 20,6 mn, énantiomère (S) 22,2 mn.
δ_{H} (400 MHz ; CDCl₃) 6,86-6,83 (2H, m, Ar), 6,80-6,75 (1H, m, Ar), 4,69-4,65 (1H, m), 4,63 (1H, d, 16,8), 4,47 (1H, d, J 16,8), 2,91-2,72 (2H, m), 2,34-2,26 (1H, m), 2,13-2,03 (1H, m).

### Exemple 7

### Synthèse de 2-chloro-1-((S)-6-fluoro-1-benzopyran-2-yl)-éthanone

On a ajouté à température ambiante 2,5 g de bromochlorométhane (18,8 mmol) à une solution de 2,0 g de (S)-6-fluoro-1-benzopyran-2-carboxylate de méthyle brut obtenu après séparation du mélange racémique correspondant par une colonne de chromatographie chirale (9,4 mmol, 87,9 % A, ee > 99 %) dans 40 ml de THF à température ambiante. On a ensuite refroidi la solution à -80/-85°C et on a lentement ajouté 7,7 ml de n-BuLi 2,5 M dans de l'hexane (19 mmol) pour maintenir la température interne à de -75°C à -80°C On a surveillé la progression de la réaction par CCM. On a ensuite acidifié la solution par addition de 2 ml d'acide acétique glacial dans 2 ml de THF à de -70°C à -80°C. On a ensuite ajouté 10 ml d'eau à -20/0°C et on a ensuite éliminé la couche aqueuse après décantation. On a concentré la couche organique sous pression réduite pour fournir le composé indiqué dans le titre (2,54 g) dans la forme d'une huile jaune (ee : 99 %, 94,8 % A).

On a ensuite facultativement purifié l'huile par chromatographie pour fournir 1,66 g de 2-chloro-1-((S)-6-fluoro-1-benzopyran-2-yl)-éthanone dans la forme d'une matière solide beige (rendement 76 %, ee : 99 %, 96,1 % A, [α]_{D} : +21°).
CHIRAL OB-H 250*4,6 mm particules 5 µm, N-heptane -IPOH (95 V-5 V), 200 nm, énantiomère (R) TR = 20,6 mn, énantiomère (S) 22,2 mn.
δ_{H} (400 MHz ; CDCl₃) 6,86-6,83 (2H, m, Ar), 6,80-6,75 (1H, m, Ar), 4,69-4,65 (1H, m), 4,63 (1H, d, J 16,8), 4,47 (1H, d, 16,8), 2,91-2,72 (2H, m), 2,34-2,26 (1H, m), 2,13-2,03 (1H, m).

### Exemple 8

### Synthèse d'un mélange de (S)-2-chloro-1-((R)-6-fluoro-1-benzopyran-2-yl)-éthanol et de (R)-2-chloro-1-((R)-6-fluoro-1-benzopyran-2-yl)-éthanol

On a refroidi une solution agitée de 2-chloro-1-((R)-6-fluoro-1-benzopyran-2-yl)-éthanone (0,7 g, 3,06 mmol, 97,6 % A) dans de l'éthanol (10 ml) à 0°C sous azote. On a ajouté NaBH₄ (152 mg, 4 mmol) à la solution et on a agité le mélange réactionnel pendant 2 h. Après avoir vérifié que le produit de départ a disparu par CPG, on a dilué le mélange avec de l'eau (17 ml) et du dichlorométhane (17 ml) et les phases se sont séparées. On a séché la strate organique sous sulfate de sodium anhydre, on l'a filtrée et concentrée sous vide pour fournir un mélange de diastéréoisomères de (S)-2-chloro-1-((R)-6-fluoro-1-benzopyran-2-yl)-éthanol et de (R)-2-chloro-1-((R)-6-fluoro-1-benzopyran-2-yl)-éthanol 55:45 (0,7 g, rendement 100 %, 97,1 % A). CHIRALPACK AD-H (250 x 4,6) mm particules 5 µm, N-heptane -IPOH (95 V-5 V), 281 nm, (S)-2-chloro-1-((R)-6-fluoro-1-benzopyran-2-yl)-éthanol : TR = 35,7 mn ; (R)-2-chloro-1-((R)-6-fluoro-1-benzopyran-2-yl)-éthanol : TR = 50,4 min.
δ_{H} (400 MHz ; CDCl₃) 6,83-6,70 (6H, m, Ar), 4,21-4,16 (1H, m), 4,02-3,96 (1H, m), 3,94-3,88 (3H, m), 3,86-3,77 (2H, m), 3,74-3,68 (1H, m), 2,97-2,74 (4H, m), 2,30-2,21 (2H, b, -OH), 2,29-2,22 (1H, m), 2,02-1,96 (2H, m), 1,89-1,78 (1H, m) ; *m*/*z* (EI) 230,050989 (M⁺ .C₁₁H₁₂ClFO₂ exige 230,05067).

### Exemple 9

### Synthèse d'un mélange de (R)-2-chloro-1-((S)-6-fluoro-1-benzopyran-2-yl)-éthanol et de (S)-2-chloro-1-((S)-6-fluoro-1-benzopyran-2-yl)-éthanol

On a refroidi à 0°C sous azote une solution agitée de 2-chloro-1-((S)-6-fluoro-1-benzopyran-2-yl)-éthanone (0,7 g, 3,06 mmol, 96,1 % A) dans de l'éthanol (10 ml). On a ajouté NaBH₄ (152 mg, 4 mmol) à la solution et on a agité le mélange réactionnel pendant 2 h. Après avoir vérifié par CPG que le produit de départ a disparu, on a dilué le mélange avec de l'eau (17 ml) et du dichlorométhane (17 ml) et les phases se sont séparées. On a séché la strate organique sous sulfate de sodium anhydre, on l'a filtrée et concentrée sous vide pour fournir un mélange de diastéréoisomères de (R)-2-chloro-1-((S)-6-fluoro-1-benzopyran-2-yl)-éthanol et de (S)-2-chloro-1-((S)-6-fluoro-1-benzopyran-2-yl)-éthanol 55:45 (0,706 g, rendement 100 %, 97,1 % A).
CHIRALPACK AD-H (250 x 4,6) mm particules 5 µm, N-heptane -IPOH (95 V-5 V), 281 nm, (R)-2-chloro-1-((S)-6-fluoro-1-benzopyran-2-yl)-éthanol : TR = 39,8 mn ; (S)-2-chloro-1-((S)-6-fluoro-1-benzopyran-2-yl)-éthanol : TR = 58,4 min.
δ_{H} (400 MHz ; CDCl₃) 6,83-6,70 (6H, m, Ar), 4,21-4,16 (1H, m), 4,02-3,96 (1H, m), 3,94-3,88 (3H, m), 3,86-3,77 (2H, m), 3,74-3,68 (1H, m), 2,97-2,74 (4H, m), 2,30-2,21 (2H, b, -OH), 2,29-2,22 (1H, m), 2,02-1,96 (2H, m), 1,89-1,78 (1H, m) ; *m*/*z* (EI) 230,050989 (M⁺ .C₁₁H₁₂ClFO₂ exige 230,05067).

### Exemple 10

### Synthèse d'un mélange de (R)-6-fluoro-2-(S)-oxyranyl-1-benzopyrane et de (R)-6-fluoro-2-(R)-oxyranyl-1-benzopyrane

On a dissous dans i-PrOH (8 ml) sous azote un mélange de (S)-2-chloro-1-((R)-6-fluoro-1-benzopyran-2-yl)-éthanol et de (R)-2-chloro-1-((R)-6-fluoro-1-benzopyran-2-yl)-éthanol 55:45 (0,7 g, 3,03 mmol, 97,1 % A) et on a refroidi le mélange réactionnel à 0-5°C. On a ajouté à la solution une solution aqueuse 2M de NaOH (4,1 ml) en 5 min et on a agité le mélange réactionnel pendant 1 h 30 min à 0-5°C puis on l'a agité pendant la nuit à température ambiante. On a ensuite dilué le mélange réactionnel avec du toluène (14 ml) et on a corrigé le pH avec de l'acide acétique (0,305 g). On a ensuite encore ajouté du toluène (14 ml) et de l'eau (1,5 ml) au mélange et les phases se sont séparées après extraction. On a ensuite lavé les phases organiques recueillies avec de l'eau (14 ml). On a ensuite rendu la phase du toluène anhydre par distillation azéotrope et on l'a concentrée jusqu'à l'état sec dans un évaporateur rotatif pour fournir un mélange de diastéréoisomères de (R)-6-fluoro-2-(S)-oxyranyl-1-benzopyrane et de (R)-6-fluoro-2-(R)-oxyranyl-1-benzopyrane 55:48 (0,56 g, rendement 95 %, 94,4 % A).
CHIRALPACK AD-H (250 x 4,6) mm particules 5 µm, N-heptane -IPOH (95 V-5 V), 281 nm, (R)-6-fluoro-2-(S)-oxyranyl-1-benzopyrane : TR = 14,4 mn ; (R)-6-fluoro-2-(R)-oxyranyl-1-benzopyrane : TR = 18,2 mn.
Diast. RR : δ_{H} (400 MHz ; CDCl₃) 6,81-6,72 (3H, m), 3,88-3,82 (1H, m), 3,21-3,17 (1H, m), 2,89-2,76 (4H, m), 2,1-2,00 (1H, m), 1,97-1,87 (1H, m) ; Diast. RS : δ_{H} (400 MHz ; CDCl₃) 6,84-6,73 (3H, m), 3,87-3,81 (1H, m), 3,15-3,10 (1H, m), 2,91-2,78 (4H, m), 2,18-2,10 (1H, m), 1,96-1,84 (1H, m)

### Exemple 11

### Synthèse d'un mélange de (S)-6-fluoro-2-(R)-oxyranyl-1-benzopyrane et de (S)-6-fluoro-2-(S)-oxyranyl-1-benzopyrane

On a dissous dans i-PrOH (8 ml) sous azote un mélange de diastéréoisomères de (R)-2-chloro-1-((S)-6-fluoro-1-benzopyran-2-yl)-éthanol et de (S)-2-chloro-1-((S)-6-fluoro-1-benzopyran-2-yl)-éthanol 55:45 (0,7 g, 3,03 mmol, 97,1 % A) et on a refroidi le mélange réactionnel à 0-5°C. On a ajouté à la solution une solution aqueuse 2M de NaOH (4,1 ml) en 5 min et on a agité le mélange réactionnel pendant 1 h 30 min à 0-5°C puis on l'a agité pendant la nuit à température ambiante. On a ensuite dilué le mélange réactionnel avec du toluène (14 ml) et on a corrigé le pH avec de l'acide acétique (0,305 g). On a ensuite encore ajouté du toluène (14 ml) et de l'eau (1,5 ml) au mélange et les phases se sont séparées après extraction. On a ensuite lavé les phases organiques recueillies avec de l'eau (14 ml). On a ensuite rendu la phase du toluène anhydre par distillation azéotrope et on l'a concentrée jusqu'à l'état sec dans un évaporateur rotatif pour fournir un mélange de diastéréoisomères de (S)-6-fluoro-2-(R)-oxyranyl-1-benzopyrane et de (S)-6-fluoro-2-(S)-oxyranyl-1-benzopyrane 52:48 (0,57 g, rendement 95 %, 94,4 % A).
CHIRALPACK AD-H (250 x 4,6) mm particules 5 µm, N-heptane -IPOH (95 V-5 V), 281 nm, (S)-6-fluoro-2-(R)-oxyranyl-1-benzopyrane : TR = 15,36 mn ; (S)-6-fluoro-2-(S)-oxyranyl-1-benzopyrane : TR = 20,32 mn.
Diast. SS : δ_{H} (400 MHz ; CDCl₃) 6,81-6,72 (3H, m), 3,88-3,82 (1H, m), 3,21-3,17 (1H, m), 2,89-2,76 (4H, m), 2,1-2,00 (1H, m), 1,97-1,87 (1H, m) ; Diast. SR : δ_{H} (400 MHz ; CDCl₃) 6,84-6,73 (3H, m), 3,87-3,81 (1H, m), 3,15-3,10 (1H, m), 2,91-2,78 (4H, m), 2,18-2,10 (1H, m), 1,96-1,84 (1H, m)

### Exemple 12

### Synthèse de 6-fluoro-2-oxyranyl-1-benzopyrane

On a ajouté à température ambiante 2,5 g de bromochlorométhane (19 mmol) à une solution de 2,0 g de 6-fluoro-1-benzopyran-2-carboxylate de méthyle brut (9,5 mmol) dans 40 ml de THF. On a ensuite refroidi la solution à -80/-85°C et on a lentement ajouté 7,8 ml de n-BuLi 2,5 M dans de l'hexane (19,5 mmol) pour maintenir la température interne à de -75°C à -80°C. On a surveillé la progression de la réaction par CCM. On a ensuite lentement ajouté une solution de BH₃ 1 M dans du THF (10 ml, 10 mmol) pour maintenir la température interne à de -70°C à -80°C. On a surveillé la progression de la réaction par CCM. On a hydrolysé le mélange par addition d'eau (15 ml) à 0°C et les phases se sont séparées. On a ensuite ajouté une solution aqueuse à 30 % d'hydroxyde de sodium à la couche organique et on a réchauffé le mélange sous reflux pendant 2 h jusqu'à ce que le 2-chloro-1-(6-fluoro-1-benzopyran-2-yl)-éthanol a disparu. On a refroidi le mélange biphasique à 20-25°C, les phases se sont séparées. On a lavé la couche organique avec de l'eau (5 ml) et on l'a concentrée jusqu'à l'état sec pour fournir du 6-fluoro-2-oxyranyl-1-benzopyrane dans la forme d'un mélange des 4 diastéréoisomères [(R,S) ; (S,R) : (R,R) ; (S,S)] = 52:48 (1,7 g, rendement 91 %).

## Revendications

1. Procédé de préparation d'un composé de formule 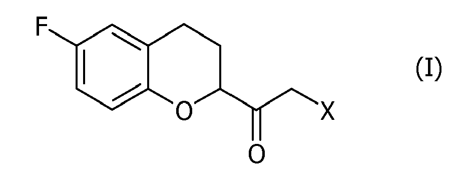 dans laquelle X est un atome d'halogène qui comprend la réaction d'un composé de formule 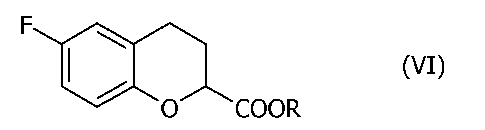 dans laquelle R est un groupe alkyle en C₁-C₆ ; avec un dérivé d'halométhyllithium de formule Li-CH₂-X dans laquelle X est tel que défini ci-dessus.

2. Procédé selon la revendication 1, **caractérisé en ce que** le dérivé d'halométhyllithium est formé dans le système réactionnel.

3. Procédé selon la revendication 2, **caractérisé en ce que** le dérivé d'halométhyllithium est formé par l'addition d'un réactif d'organolithium avec un dihalométhane.

4. Procédé selon la revendication 3, **caractérisé en ce que** le dihalométhane est le bromochlorométhane.

5. Procédé selon la revendication 3 ou 4, **caractérisé en ce que** le réactif d'organolithium est le n-butyllithium.

6. Procédé selon l'une des revendications 3 à 5, **caractérisé en ce que** le composé de la formule (VI), le dihalométhane et le réactif d'organolithium sont utilisés dans un rapport molaire d'environ 1:2:2.

7. Procédé selon l'une quelconque des revendications précédentes, **caractérisé en ce que** le dérivé d'halométhyllithium est le chlorométhyllithium.

8. Procédé selon l'une quelconque des revendications précédentes, **caractérisé en ce que** la réaction d'un composé de formule VI avec un dérivé d'halométhyllithium est réalisée en présence d'un solvant.

9. Procédé selon la revendication 8, **caractérisé en ce que** le solvant est le tétrahydrofurane (THF).

10. Procédé selon la revendication 1, **caractérisé en ce que** l'addition de l'halométhyllithium au composé de formule (VI) est réalisée à une température dans l'intervalle de -100°C à 0°C.

11. Procédé selon la revendication 10, **caractérisé en ce que** l'addition de l'halométhyllithium est réalisée à une température dans l'intervalle de -85°C à -50°C.

12. Procédé de préparation d'un composé de formule 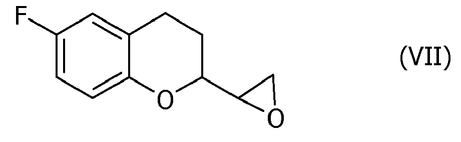 qui comprend une conversion d'un composé de formule (VI) en un composé de la formule (I) selon le procédé de la revendication 1.

13. Procédé selon la revendication 12, qui comprend en outre la réduction d'un composé de formule I pour fournir un composé de formule (VIII) : 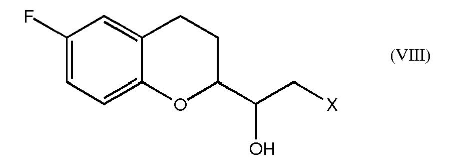 dans laquelle X est un atome d'halogène,
et la réaction dudit composé de formule (VIII) avec une base pour fournir le composé époxyde de formule (VII).

14. Procédé de préparation d'un composé de formule 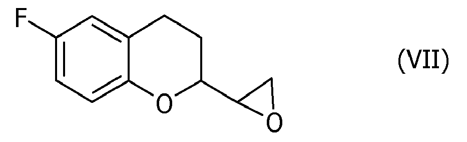 qui comprend la réaction d'un composé de formule 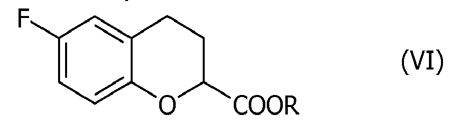 dans laquelle R est un groupe alkyle en C₁-C₆ ; avec un dérivé d'halométhyllithium de formule Li-CH₂-X dans laquelle X est un atome d'halogène, pour fournir un mélange réactionnel ; la réduction *in situ* dudit mélange réactionnel ; et la cyclisation en présence d'une base.

15. Procédé de préparation du nebivolol qui comprend la conversion d'un composé de formule (VI) en un composé de formule (I) selon le procédé de la revendication 1.

16. Procédé de préparation du nebivolol qui comprend une conversion de type « one-pot » d'un composé de formule (VI) en un composé de formule (VII) selon le procédé de la revendication 14.

## Patentansprüche

1. Verfahren zur Herstellung einer Verbindung der Formel 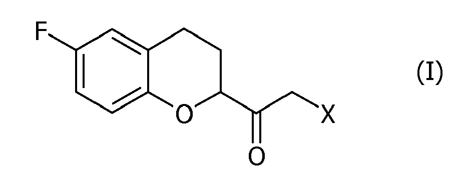 in welcher X ein Halogenatom ist, wobei das Verfahren das Reagieren einer Verbindung der Formel 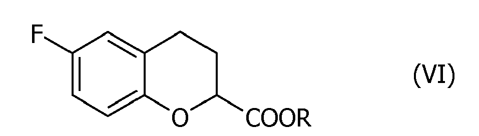 in welcher R eine C₁-C₆-Alkylgruppe ist, mit einem Halogenmethyllithium-Derivat der Formel Li-CH₂-X, in welcher X der obigen Begriffsbestimmung entspricht, umfasst.

2. Verfahren nach Anspruch 1, **dadurch gekennzeichnet, dass** das Halogenmethyllithium-Derivat in dem Reaktionssystem gebildet wird.

3. Verfahren nach Anspruch 2, **dadurch gekennzeichnet, dass** das Halogenmethyllithium-Derivat gebildet wird, indem ein Organolithiumreagenz mit einem Dihalogenmethan zugesetzt wird.

4. Verfahren nach Anspruch 3, **dadurch gekennzeichnet, dass** es sich bei dem Dihalogenmethan um Bromchlormethan handelt.

5. Verfahren nach Anspruch 3 oder 4, **dadurch gekennzeichnet, dass** es sich bei dem Organolithiumreagenz um n-Butyllithium handelt.

6. Verfahren nach einem der Ansprüche 3 bis 5, **dadurch gekennzeichnet, dass** die Verbindung der Formel (VI), das Dihalogenmethan und das Organolithiumreagenz in einem Molverhältnis von ungefähr 1:2:2 verwendet werden.

7. Verfahren nach einem beliebigen der vorhergehenden Ansprüche, **dadurch gekennzeichnet, dass** es sich bei dem Halogenmethyllithium-Derivat um Chlormethyllithium handelt.

8. Verfahren nach einem beliebigen der vorhergehenden Ansprüche, **dadurch gekennzeichnet, dass** die Reaktion einer Verbindung der Formel VI mit einem Halogenmethyllithium-Derivat in Gegenwart eines Lösemittels erfolgt.

9. Verfahren nach Anspruch 8, **dadurch gekennzeichnet, dass** es sich bei dem Lösemittel um Tetrahydrofuran (THF) handelt.

10. Verfahren nach Anspruch 1, **dadurch gekennzeichnet, dass** der Zusatz des Halogenmethyllithiums zur Verbindung der Formel (VI) bei einer Temperatur innerhalb des Intervalls von -100 °C bis 0 °C erfolgt.

11. Verfahren nach Anspruch 10, **dadurch gekennzeichnet, dass** der Zusatz des Halogenmethyllithiums bei einer Temperatur innerhalb des Intervalls von -85 °C bis -50 °C erfolgt.

12. Verfahren zur Herstellung einer Verbindung der Formel 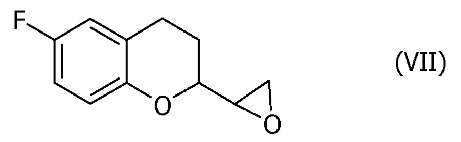 wobei dieses eine Umwandlung einer Verbindung der Formel (VI) in eine Verbindung der Formel (I) gemäß dem Verfahren des Anspruchs 1 umfasst.

13. Verfahren nach Anspruch 12, welches darüber hinaus die Reduktion einer Verbindung der Formel I umfasst, um eine Verbindung der Formel (VIII) zu erhalten: 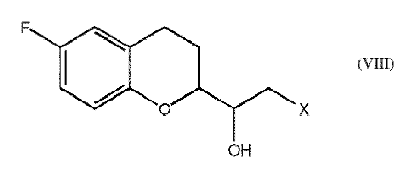 in welcher X ein Halogenatom ist,
sowie die Umsetzung der Verbindung der Formel (VIII) mit einer Base, um die Epoxidverbindung der Formel (VII) zu erhalten.

14. Verfahren zur Herstellung einer Verbindung der Formel 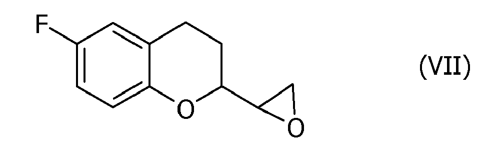 wobei im Rahmen des Verfahrens eine Verbindung der Formel 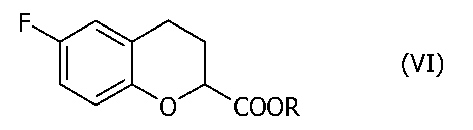 in welcher R eine C₁-C₆-Alkylgruppe ist, mit einem Halogenmethyllithium-Derivat der Formel Li-CH₂-X, in welcher X ein Halogenatom ist, umgesetzt wird, um eine Reaktionsmischung zu erhalten; die Reaktionsmischung einer *in-situ-*Reduktion unterzogen wird; und ein Ringschluss in Gegenwart einer Base erfolgt.

15. Verfahren zur Herstellung von Nebivolol, wobei dieses eine Umwandlung einer Verbindung der Formel (VI) in eine Verbindung der Formel (I) gemäß dem Verfahren des Anspruchs 1 umfasst.

16. Verfahren zur Herstellung von Nebivolol, wobei dieses eine Umwandlung einer Verbindung der Formel (VI) in eine Verbindung der Formel (I) gemäß dem Verfahren des Anspruchs 14 in einer Eintopfreaktion umfasst.

## Claims

1. A process for preparing a compound of formula 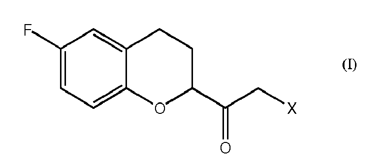 wherein X is a halogen atom,
which comprises reacting a compound of formula 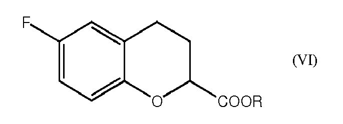 wherein R is a (C₁-C₆)-alkyl group,
with a halomethyllithium derivative of formula Li-CH₂-X in which X is as defined above.

2. The process according to claim 1, wherein the halomethyllithium derivative is formed in the reaction system.

3. The process according to claim 2, wherein the halomethyllithium derivative is formed by the addition of an organolithium reagent to a dihalomethane.

4. The process according to claim 3, wherein the dihalomethane is bromochloromethane.

5. The process according to claim 3 or claim 4, wherein the organolithium reagent is n-butyllithium.

6. The process according to any of claims 3 to 5, wherein the compound of formula (VI), the dihalomethane and the organolithium reagent are used in a molar ratio of about 1:2:2.

7. The process according to any preceding claims, wherein the halomethyllithium derivative is chloromethyllithium.

8. The process according to any preceding claims, wherein the reaction of a compound of formula VI with a halomethyllithium derivative is carried out in the presence of a solvent.

9. The process according to claim 8, wherein the solvent is tetrahydrofuran (THF).

10. The process according to claim 1, wherein the addition of the halomethyllithium to the compound of formula (VI) is carried out at a temperature in the range from -100°C to 0°C.

11. The process according to claim 10, wherein the addition of the halomethyllithium is carried out at a temperature in the range from -85°C to -50°C.

12. A process for preparing a compound of formula 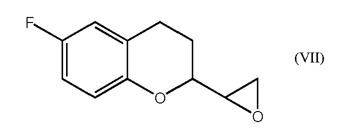 which comprises converting a compound of formula (VI) into a compound of formula (I) by the process of claim 1.

13. The process according to claim 12, further comprising
reducing a compound of formula I to give a compound of formula (VIII): 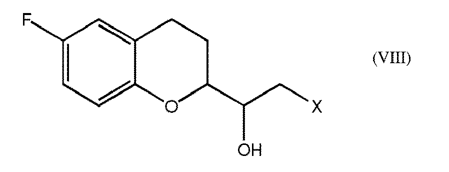 in which X is a halogen atom, and
reacting said compound of formula (VIII) with a base to give the epoxide compound of formula (VII).

14. A process for preparing a compound of formula 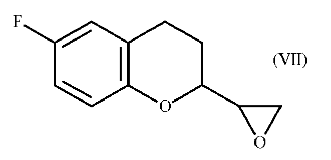 which comprises reacting a compound of formula 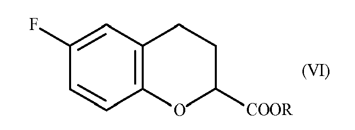 in which R is a (C₁-C₆)-alkyl group, with a halomethyllithium derivative of formula Li-CH₂-X in which X is a halogen atom, to give a reaction mixture;
*in situ* reducing said reaction mixture; and
cyclizing in the presence of a base.

15. A process for the preparation of nebivolol which comprises converting a compound of formula (VI) into a compound of formula (I) by the process of claim 1.

16. A process for the preparation of nebivolol which comprises a one-pot conversion of a compound of formula (VI) into a compound of formula (VII) by the process of claim 14.
